(19) Europäisches Patentamt

European Patent Office

Office européen des brevets

(11) EP 0 975 362 B1

(12) FASCICULE DE BREVET EUROPEEN

(45) Date de publication et mention de la délivrance du brevet:
**30.07.2003 Bulletin 2003/31**

(21) Numéro de dépôt: **98909232.5**

(22) Date de dépôt: **05.03.1998**

(51) Int Cl.[7]: **A61K 39/108**, A61K 39/00, A61K 39/35 // (A61K39/108, 39:00), (A61K39/108, 39:00), (A61K39/35, 39:108)

(86) Numéro de dépôt international:
**PCT/BE98/00030**

(87) Numéro de publication internationale:
**WO 98/039029 (11.09.1998 Gazette 1998/36)**

(54) **COMPOSITION PHARMACEUTIQUE OU ALIMENTAIRE POUR LE TRAITEMENT DE PATHOLOGIES LIEES A UN REJET DE GREFFE, UNE REACTION ALLERGIQUE OU AUTO-IMMUNE**

PHARMAZEUTISCHE ODER NAHRUNGSMITTELZUSAMMENSETZUNG FÜR DIE BEHANDLUNG VON PATHOLOGIEN DIE ASSOZIIERT SIND MIT EINEM TRANSPLANTATABSTOSS, EINER ALLERGISCHEN ODER AUTOIMMUNREAKTION

PHARMACEUTICAL OR FOOD COMPOSITION FOR TREATING PATHOLOGIES RELATED TO GRAFT VERSUS HOST, ALLERGIC OR AUTOIMMUNE REACTION

(84) Etats contractants désignés:
**AT BE CH DE DK ES FR GB IT LI NL SE**

(30) Priorité: **05.03.1997 BE 9700199**

(43) Date de publication de la demande:
**02.02.2000 Bulletin 2000/05**

(73) Titulaire: **BioTech Tools S.A.**
**1120 Bruxelles (BE)**

(72) Inventeurs:
• **DUCHATEAU, Jean**
**B-1050 Bruxelles (BE)**
• **SERVAIS, Geneviève**
**B-7060 Horrues (BE)**

(74) Mandataire: **Schreiber, Christoph, Dr. et al**
**Patentanwälte von Kreisler Selting Werner,**
**Postfach 10 22 41**
**50462 Köln (DE)**

(56) Documents cités:
**WO-A-94/29459**  **WO-A-95/24923**
**WO-A-97/06821**  **WO-A-98/23735**

• **STEPHANIE KÖNEN-WAISMAN ET AL.: "SELF AND FOREIGN 60-KILODALTON HEAT SHOCK PROTEIN T CELL EPITOPE PEPTIDES SERVE AS IMMUNOGENIC CARRIERS FOR A T CELL-INDEPENDENT SUGAR ANTIGEN." JOURNAL OF IMMUNOLOGY., vol. 154, no. 11, 1995, pages 5977-5985, XP002083262 BALTIMORE US**
• **Sondermann et al., Biol. Chem, 2000, Vol. 381 (12), pp. 1165-1174, résumé**
• **Becker et al., J. Cell. Biol., 2002, Vol. 158(7), pp. 1277-85, résumé**

Remarques:
Le dossier contient des informations techniques présentées postérieurement au dépôt de la demande et ne figurant pas dans le présent fascicule.

**Description**

Objet de l'invention

La présente invention est relative à une nouvelle composition pharmaceutique ou alimentaire destinée au traitement de pathologies liées à un rejet de greffe, une réaction allergique ou auto-immune.

Arrière-plan technologique à la base de l'invention

Depuis une douzaine d'années, des études contrôlées décrivent la désensibilisation reposant sur l'administration orale d'allergènes (1). Cette méthode se fonde sur le fait que l'administration orale d'un antigène facilite l'acquisition d'une tolérance immunologique à son égard. La voie digestive constitue le mode de contact de l'organisme avec des antigènes, d'origine alimentaire ou microbienne. Cependant, les réactions allergiques sont rares. L'administration per os de globules rouges de mouton (GRM) à des rats empêche ceux-ci de produire plus tard des anticorps anti-GRM après une injection sous-cutanée, alors que sans prise orale préalable, la réponse allergique eut été présente. Ce phénomène constitue immunologiquement ce que l'on appelle la tolérance orale.

Cette méthode de désensibilisation per os a été validée dans des études prospectives et contrôlées, et permet de réduire les risques d'anaphylaxie, en particulier. pour le pollen de bouleau et les acariens. Elle est déjà accessible sur le marché des vaccins par une présentation sous forme buvable (vendue par la société Laboratoire des Stallergènes - Paris).

Par ailleurs, on peut considérer que le bénéfice en terme de protection de l'allergie au lait des nourrissons qui est constaté depuis l'introduction de nouvelles formules de laits en poudre prédigérés enzymatiquement résulterait de l'induction de tolérances immunologiques par la présentation des antigènes sous forme de peptides.

Cependant, il est difficile de prédire ou constater l'efficience de la désensibilisation. L'observation clinique permet, après coup, de constater ou non l'amélioration éventuelle des symptômes.

Il est connu par la demande de brevet internationale WO96/36880 de pouvoir détecter et/ou quantifier des ligands spécifiques d'une pathologie liée à une réponse allergique, auto-immune ou du cancer du poumon, par un test de compétition entre des ligands présents dans un échantillon avec d'autres ligands discriminables. Ce test se base sur le fait que les sujets allergiques et symptomatiques reconnaissent par leurs anticorps des épitopes différents de ceux reconnus par les anticorps de sujets tolérants sur la même structure antigénique spécifique de ladite pathologie. Ce document décrit en outre la possibilité de mesurer l'évolution de cette spécificité, en particulier dans le cas d'enfants allergiques au lait, et l'évolution vers l'acquisition in vivo de la tolérance au lait.

Le document WO-A-97/06821 décrit des compositions comprenant une protéine de stress et un épitope d'une structure antigénique incorporés dans des véhicules permettant l'absorption ou l'injection desdites compositions par une application mucosale ou cutanée.

Dans le troisième paragraphe de la page 10, ce document décrit que lesdites structures antigéniques peuvent être associées à des maladies auto-immunes ou de l'allergie, et que dans ce cas, ledit antigène est administré en combinaison avec lesdites protéines de stress dans une quantité suffisante pour être tolérogéniques ou pour inhiber une réponse immunitaire préexistante à l'encontre dudit antigène dans un sujet.

Il est indiqué que la quantité de protéines de stress requise pour inhiber cette réponse immunitaire est supposée substantiellement plus importante que les quantités pour obtenir une stimulation.

Cependant, aucun exemple d'application de telles compositions n'est décrit dans ce document. En particulier, aucune démonstration de l'application d'une telle composition *in vivo* n'est donnée dans ce document. En outre, aucune dose d'administration desdites compositions n'est ni décrite, ni suggérée, dans ce document.

Le document WO-A-95/24920 décrit l'utilisation de complexes constitués de l'association d'une protéine de stress et d'un peptide en tant que vaccin prophylactique ou thérapeutique contre des pathogènes intracellulaires.

Ce document ne décrit nullement la possibilité d'utiliser de tels complexes *in vivo* dans le traitement des maladies auto-immunes, de l'allergie ou des rejets de greffes.

Buts de l'invention

La présente invention a pour but de fournir une nouvelle composition qui peut être de type pharmaceutique ou alimentaire visant à modifier la réponse immunitaire de patients vis-à-vis d'une pathologie liée à une réaction allergique, auto-immune ou vis-à-vis de phénomènes de rejet de greffe, de manière à ce que la réponse immunitaire desdits patients se rapproche de la tolérance naturelle manifestée par des sujets normaux (qui demeurent asymptomatiques bien que susceptibles d'être exposés aussi à cette pathologie).

La présente invention vise également à fournir une composition pharmaceutique ou alimentaire de faible coût, dont l'administration soit aisée et qui puisse être utilisée de manière prophylactique et/ou thérapeutique.

<u>Éléments caractéristiques de l'invention</u>

La présente invention concerne une composition pharmaceutique ou alimentaire comprenant un véhicule pharmaceutique ou alimentaire adéquat, une protéine de stress d'une bactérie saprophyte (dénommée également "heat shock protein" ou HSP) et au moins un des épitopes (conformationnel ou séquentiel) d'une structure antigénique, ladite structure antigénique induisant un rejet de greffe, une réaction allergique ou une réaction auto-immune. De préférence, le véhicule pharmaceutique ou alimentaire de la composition est adéquat pour une administration par voie mucosale (notamment par voie orale) ou cutanée.

Avantageusement, la protéine de stress et l'épitope forment un complexe de manière naturelle (c'est-à-dire sans formation de lien covalent) comme décrit par Roamn et al., Febs (1994), Fouri et al., The Journal of Biological Chemistry, Volume 269 n° 48, pp. 30470-30478 (1994), Palleros et al., The Journal of Biological Chemistry, Volume 269 n° 48, pp. 13107-13114 (1994), Grageroov et Gottesman, Journal of Molecular Biology, n° 241, pp. 133-135 (1994), et Schmid et al., Science, Volume 260, p. 1991 (1994).

Selon l'invention, l'épitope (dénommé également déterminant antigénique) est obtenu par une hydrolyse de préférence enzymatique, notamment à la pepsine, de ladite structure antigénique.

La protéine de stress est une protéine bactérienne de stress, présente dans les bactéries saprophytes telles que E. coli.

Parmi les protéines de stress de la présente invention, on peut mentionner la protéine de stress GroEL, les protéines de stress GrpE, DnaK ou DnaJ telles que notamment décrites par Hendrick et Hartl (Annual Review of Biochemistry, n° 62, p. 349 (1993)) ou les heat shock proteins HSP 60, 70, ...

On entend par "phénomène de rejet de greffe, réaction allergique ou auto-immune", les réactions d'hypersensibilité de type immédiat ou différé provoquées pour le contact notamment avec un allergène (cette réaction peut être immédiate et spécifique (anaphylaxie, urticaire, etc.) ou différée dans le temps) ou les maladies auto-immunes et les désordres du système immunitaire de type immédiat ou différé liés aux rejets de greffes de type hôte contre greffe et greffe contre hôte.

L'auto-immunité est un état d'immunisation d'un sujet contre ses propres constituants et le phénomène de rejet de greffe est un état d'immunisation d'un sujet contre des constituants étrangers (fluides corporels tels que sang, liquide céphalo-rachidien, ..., cellules, tissus, organes, anticorps, ...) implantés de manière volontaire chez le patient. Ces phénomènes sont en particulier observés dans les pathologies choisies parmi le groupe constitué par les infections liées au SLE (Systemic Lupus Erythematosus disease), le syndrome Gougerot-Sjögren (ou pathologie Sjögren), la polyarthrite rhumatoïde, ainsi que les pathologies du type sarcoïdosis et osteopenia, la spondylarthrite, la scleroderma, la sclérose en plaques (multiple sclerosis), la sclérose amyotrophique latérale, l'hyperthyroïdisme, la maladie d'Addison, l'anémie hémolytique auto-immune, la maladie de Crohn, le syndrome de Goddpasture, la maladie de Graves, la thyroïdite d'Hashimoto, l'hémorragie purpurale idiopathique, les diabètes insulino-dépendants, la myasthénie, le pemphigus vulgaris, l'anémie pernicieuse, le glomerulonephritis poststreptococcal, le psoriasis et la stérilité spontanée, ainsi que les phénomènes immédiats ou différés observés lors de rejets de greffe.

On entend par "structure antigénique induisant un rejet de greffe, une réaction allergique ou auto-immune", des allergènes, de préférence choisis parmi le groupe constitué par les antigènes majeurs allergiques présents dans les aliments tels que les oeufs, le soja, le lait, en particulier la bêta-lactoglobuline bovine (BLG), issue du lait de vache, les antigènes majeurs allergiques présents dans les végétaux, les moisissures, les médicaments (en particulier les antibiotiques), les pollens, les antigènes majeurs allergiques présents dans les animaux, en particulier dans les poils, le venin, en particulier le venin de guêpe, les antigènes majeurs de la réaction allergique aux acariens, à la mite présente dans la poussière de maison (antigène P1 Dermatophagoïdes pteronyssinus), l'antigène majeur de l'Aspergillus fumagatus, et l'entérotoxine B staphylococcale (SEB).

D'autres exemples non limitatifs d'allergènes ou mélanges d'allergènes ont également été décrits dans la publication ISBN-91-970475-5-4 de Pharmacia AB.

La "structure antigénique" peut également être un complexe antigénique induisant une maladie auto-immune. De préférence, cette structure antigénique est spécifique du lupus (SLE) ou de la pathologie de Sjôgren, en particulier la membrane plasmatique ou une portion de cette membrane contenant du DNA membranaire ayant un poids supérieur à 100 KD, tel que notamment décrit dans la demande de brevet WO96/1372 dont le numéro de publication.

D'autres exemples non limitatifs de complexes antigéniques induisant des maladies auto-immunes ont également été décrits par Roitt I. M. (Essential Immunology, Blackwell Scientific Publication (ch. 14) ISBN 0-632-01994-8) et par Humbel R. L. (Auto-anticorps et maladies auto-immunes, Ed. Scientifiques Elsevier (1994) ISBN 2-906077-58-5).

Cette structure antigénique peut également être un locus majeur d'histocompatibilité (MHC I et/ou MHC II) ou une portion de celui-ci spécifique d'un individu et intervenant dans les phénomènes de rejet de greffes (y compris les transfusions de fluides corporels).

Le véhicule pharmaceutique ou alimentaire adéquat selon la présente invention peut être n'importe quel additif ou support, tel qu'une substance compatible non toxique pour l'administration de la composition selon l'invention à un patient. Le type de véhicule pharmaceutique ou alimentaire adéquat utilisé dépendra du mode d'administration choisi. En particulier, pour l'administration orale, ceux-ci peuvent consister en des solutions aqueuses, des sirops, des tablettes, des capsules, etc. D'autres véhicules pharmaceutiques tels que des crèmes ou des onguents peuvent être choisis en fonction du type d'administration, en particulier pour des administrations cutanées.

L'homme du métier peut également adapter le véhicule pharmaceutique en fonction d'une administration subcutanée, intradermale, intraveineuse, intramusculaire, parentérale, par voie d'inhalation nasale ou buccale, etc.

Le pourcentage de composé actif présent dans la composition selon l'invention dépendra du type de patient et de pathologie traité et de la voie d'administration. Les doses seront uniquement limitées par la tolérance du produit par le patient, ainsi que par les fréquences d'administration.

Les concentrations d'administration seront en particulier choisies de manière à ce que les signes et symptômes des pathologies susmentionnées soient réduits, de préférence supprimés, par les doses d'administration prévues par la posologie.

De manière inattendue, les Inventeurs ont découvert que l'utilisation de la composition pharmaceutique et/ou alimentaire selon l'invention permet de modifier la réponse immunitaire d'un patient induite par ladite structure antigénique. La modification de la réponse immunitaire d'un patient peut notamment être détectée et quantifiée selon le procédé et la technique décrits dans la demande de brevet WO96/36880 ou par toute méthode d'analyse clinique du patient traité (y compris de manière prophylactique) bien connue de l'homme du métier.

Un autre aspect de la présente invention concerne l'utilisation de la composition selon l'invention pour la préparation d'un médicament destiné à modifier la réponse immunitaire d'un patient vis-à-vis d'une structure antigénique induisant un rejet de greffe, une réaction allergique ou auto-immune. En particulier, la présente invention concerne l'utilisation de la composition pharmaceutique et/ou alimentaire selon l'invention pour la préparation d'un médicament destiné à la désensibilisation d'allergies atopiques ou non atopiques.

Un autre aspect de la présente invention concerne l'utilisation de la composition pharmaceutique et/ou alimentaire selon l'invention pour la préparation d'un médicament destiné à la prévention ou au traitement des réactions allergiques, des maladies auto-immunes susmentionnées, au traitement ou à la prévention de rejets de greffe, éventuellement en combinaison avec un produit spécifique pour diminuer ou neutraliser les réactions allergiques, les réactions auto-immunes et les phénomènes de rejet de greffe (en particulier l'administration d'immunosuppresseurs tels que l'azathioprine, les stéroïdes, les globulines anti-lymphocitaires, la cyclosporine A, la rapamycine, le KF-506 (tacrolimus) ou des lymphokines (en particulier l'IL-10), leurs analogues et leurs agonistes bien connus de l'homme du métier.

On entend par "analogues et agonistes" de ces molécules, d'autres molécules, ou des dérivés de. ces molécules, agissant sur le même récepteur ou via le même mécanisme d'action que les produits spécifiques susmentionnés.

La présente invention concerne également un procédé de traitement thérapeutique ou prophylactique d'un patient comprenant l'étape d'administration de la composition selon l'invention audit patient de manière à modifier la réponse immunitaire du patient vis-à-vis d'une structure antigénique induisant un rejet de greffe, une réaction allergique ou auto-immune.

La présente invention sera décrite de manière plus détaillée en référence aux figures décrites dans les exemples d'exécution ci-dessous.

Exemples

A. Base du modèle considéré

*a) Utilisation de la voie orale*

L'administration orale permet une induction de tolérances immunologiques, et est appliquée de façon de plus en plus étendue dans le domaine de la désensibilisation anti-allergique. Elle demande cependant l'usage de quantités plus importantes d'antigènes que par voie parentérale, et doit s'étendre sur des périodes d'au moins plusieurs années (2, 3). L'optimalisation du régime des doses administrée et de leur périodicité est adaptable par l'homme du métier de manière à éviter les réactions syndromiques (réplication de la symptomatologie allergique en cas de surdosage), qui sont fréquentes mais non dangereuses en raison de la progressivité lente de l'augmentation des doses administrées (2).

*b) Utilisation de complexes peptides-protéines de stress*

Les protéines de stress (heat shock proteins (HSP)) constituent une série de familles de protéines, très bien conservées durant l'évolution depuis les bactéries jusqu'à l'homme, et qui ont la capacité de se lier aux peptides ou aux protéines dont la structure conformationnelle est altérée ou en voie de conformation définitive (4).

**EP 0 975 362 B1**

Elles ont plusieurs rôles dont la participation au transport intracellulaire menant à l'assemblage polypeptidique pour la synthèse de certaines protéines ou leur élimination. Certaines sont exprimées à la surface de différentes cellules et peuvent contribuer à la présentation antigénique, en particulier à des lymphocytes T aux récepteurs pour antigène de type gamma-delta, qui colonisent les muqueuses et les organes lymphoïdes associés à la muqueuse digestive.

La présentation antigénique par l'intermédiaire des HSP de la famille HSP70, aux lymphocytes T gamma-delta (γ, δ) permet de se passer de la présentation dépendante du complexe majeur d'histocompatibilité de type II.

L'injection parentérale à des animaux d'expérience de complexes HSP-peptides permet d'obtenir un effet adjuvant remarquable (5, 6) déterminant ou amplifiant le pouvoir antigénique de ces peptides.

Certaines HSP de bactéries des familles HSP60 et HSP70 sont la cible de réponses immunes qui ont un rôle protecteur à l'égard de l'infection par ces germes.

Il a été proposé récemment de désensibiliser par voie orale en donnant des extraits peptidiques d'E. Coli contenant de la HSP60, à des patients atteint d'arthrite rhumatoïde, avec un certain effet bénéfique (5, 6). Considérant le peu d'effet secondaire, les auteurs proposent de tenter l'essai sur d'autres affections inflammatoires pour manipuler une réponse dirigée contre l'une de ces HSP microbiennes elle-même, considérée comme substitut d'un auto-antigène.

De manière inattendue, les Inventeurs ont découvert que les protéines de stress constituaient un vecteur remarquable pour présenter des peptides au systèmes lymphoïde du tube digestif et induire une tolérance. Les protéines de stress des bactéries saprophytes semblent être les plus abondantes naturellement dans la lumière digestive. Il est probable aussi que les peptides issus de la digestion alimentaire constituent la masse la plus abondante des fragments antigéniques disponibles à la formation de complexes HSP-peptides. Cependant, l'abondance des peptides générés, et la quantité présumée limitée des HSP bactériennes rend aléatoire la formation d'une quantité immunologiquement efficiente de ces complexes HSP-peptides antigéniques, et ceci d'autant que la quantité absorbée d'antigène à visée désensibilisante est très faible (quelques dizaines de µg), en regard de la charge protéique alimentaire.

Les Inventeurs ont proposé de favoriser la formation de ces complexes avant l'arrivée dans le tube digestif, c'est à dire in vitro, en utilisant des protéines de stress de E. Coli purifiées et des peptides issus de la digestion pepsinique de la BLG.

*c) Utilisation de tests de compétition entre anticorps sériques et monoclonaux pour la BLG*

Les deux anticorps monoclonaux dénommés ci-après M6 et M7 reconnaissent chacun un épitope conformationnel différent sur la molécule de BLG. On utilise leurs propriétés qualitatives différentes comme marqueur de reconnaissance d'épitopes singuliers dans une compétition avec l'ensemble des anticorps sériques d'un sujet. Il ressort d'études cliniques que les sujets symptomatiques et les sujets asymptomatiques reconnaissent sur cette molécule des épitopes qui pour une part au moins, sont différents (7), ce que l'on dénomme ci-dessous les profils épitopiques.

L'épitope reconnu par M6 est particulièrement bien reconnu par les sujets allergiques et symptomatiques. La fixation de l'anticorps M6 sur la BLG intacte est en effet mieux inhibée par les sérums d'enfants allergiques au lait que ceux de sujets non allergiques, qu'il s'agisse d'enfants ou d'adultes en bonne santé (donneurs de sang).

L'épitope reconnu par M7 est mieux reconnu par les sujets asymptomatiques que par les sujet allergiques. La fixation de l'anticorps M7 sur la BLG est mieux inhibée par les sujets asymptomatiques que par les sujets allergiques.

On utilise la compétition de liaison antigénique contre M6 comme indice de spécificité représentant du profil d'épitopes reconnu par les sujets allergiques, et complémentairement la compétition contre M7 comme indice de spécificité représentant du profil d'épitopes reconnu par les sujets asymptomatiques.

La validation de cette interprétation a été confirmée longitudinalement par des études cliniques. L'acquisition d'un état de tolérance au lait s'accompagne d'une conversion de la spécificité fine des anticorps sériques, vers le profil typique des sujets asymptomatiques.

C'est cette discrimination d'épitopes exprimée à l'échelon des anticorps circulants qui sert ici d'outil d'analyse pour l'influence de la modulation antigénique orale.

B. Modèle expérimental

Des souris syngéniques ont reçu, dans leur eau de boisson, de très faibles quantités de peptides issus de la digestion pepsinique de bêta-lactoglobuline (BLG) préalablement couplés ou non avec des protéines de stress purifiées et dont la capacité fonctionnelle était intacte (capacité de se lier à des peptides ou des protéines altérées).

*a) Origine animale et conditions d'élevage*

40 individus de 8 à 16 semaines ont été prélevés dans un élevage de souris Balbc soumises depuis plusieurs générations à une alimentation pauvre en lait de vache : 30 µg de bêta-lactoglobuline / gramme de granulés nutritifs.

**5**

*b) Préparation des complexes antigéniques*

La BLG a été digérée au contact de pepsine couplée à de l'agarose (Sigma) dans des conditions de digestion incomplète, puis filtrée sur filtre de 10000 Daltons. La concentration du produit de digestion (pB) a été mesurée par spectrophotométrie (rendement de 30 à 50% de la protéine intacte).

Des solutions en tampon phosphate (PBS) de 1 µg/ml ont été incubées avec des solutions de 1 µg/ml des protéines de stress des E. Coli suivantes : DnaK, DnaJ, GroEL, GrpE (Stressgen) pendant une heure au moins à température ordinaire. Des aliquots de 1 ml de chaque combinaison ont été congelés.

*c) Groupes de traitement et posologie des complexes par voie orale*

Une solution de complexes (1 ml) a été ajoutée, après dégel, au biberon d'eau de 100 ml quotidiennement distribué pour chaque cage de quatre souris. Chaque type de complexe est administré à 8 souris. Un groupe contrôle reçoit de l'antigène pB non complexé.

La solution a été ajoutée 3 fois par semaine durant deux semaines (soit six fois), à partir du temps zéro.

*d) Réponse anticorps*

Un prélèvement individuel de sang a été réalisé dans le plexus rétro-orbitaire au temps zéro et après 4 semaines. Les animaux sont anesthésiés à l'éther puis exsanguinés, au bout de 8 semaines.

La spécificité des anticorps sériques a été examinée par un test de compétition de type ELISA.

*e) Test de spécificité d'anticorps par compétition*

Des plaques multipuits en polystyrène sont passivement recouvertes, par absorption à température ordinaire, d'une faible quantité de BLG (0,3 µg/ml en tampon bicarbonate), puis saturées par de la gélatine (1%, poids/vol - Haemacel (R)).

Le sérum de souris est dilué au 1/100 en tampon de dilution (PBSdil) constitué de : PBS-EDTA (10 mM) - Tween 20 (0,05%) - gélatine (Haemacel-1%).

Deux anticorps monoclonaux de souris produits ont été sélectionnés pour leur spécificité à l'égard d'épitopes conformationnels de la BLG. Ils ont été biotinés et sont utilisés à leur dilution limite de fixation antigénique définie de la façon suivante : celle qui permet un signal maximal mais sensible à toute réduction de la charge en antigène, à sa propre dilution, et inhibable par la compétition avec un pool de sérums de souris non traitées. Celles-ci produisent en effet des anticorps naturels contre la BLG, en relation avec l'exposition antigénique alimentaire, même minime.

100 µl de sérum dilué et d'anticorps biotiné sont mélangés dans un puits, en double exemplaire.

Après une incubation d'une nuit à température ordinaire, la fixation de l'anticorps monoclonal est mesurée par la rétention proportionnelle de biotine révélée par captation de streptavidine couplée à de la peroxydase de raifort. Celle-ci colore un substrat d'orthophénylène diamine. La densité optique (D.O.) est mesurée par spectrophotométrie. Le bruit de fond (b.f) est mesuré dans des puits sans antigène. La fixation maximale est définie soit en l'absence de compétition (anticorps monoclonal seul), soit en présence de sérum particulièrement peu inhibiteur.

Les résultats sont exprimés en pourcentage de l'inhibition de fixation de l'anticorps monoclonal par :

$$\% \text{ inhib} = 100 \times (\text{D.O. du test} - \text{D.O. b.f.}) / (\text{D.O. maximale} - \text{D.O. b.f.})$$

La correspondance entre profil d'épitopes reconnus sur l'antigène et l'état clinique du sujet (tolérance ou pas) est confirmée par d'autres exemples :

- modèle d'allergie aux acariens :

  l'évolution de la spécificité fine des anticorps anti-acariens chez des enfants allergiques montre l'existence d'un profil d'épitopes sous l'effet de la désensibilisation induite tant par voie parentérale qu'orale,

- l'évolution des anticorps

*g) Résultats*

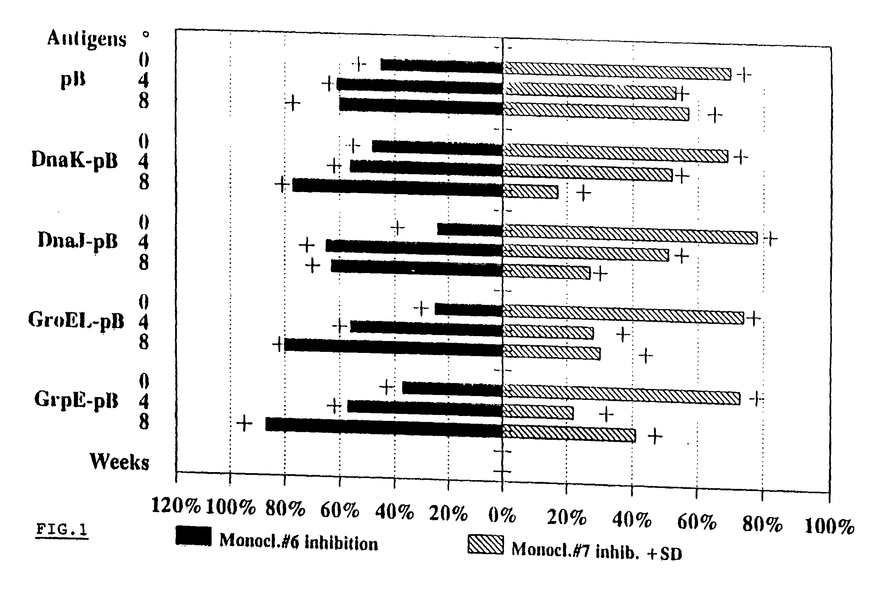

La figure 1 résume les données de l'expérimentation :

*Inhibition de l'anticorps M6*

Sur la partie gauche, sont représentées les évolutions des moyennes d'inhibition (+ écart type) de la fixation de l'anticorps monoclonal M6 par les sérums individuels, pour les différents groupes de traitement.

Les données chiffrées sont reprises dans le tableau 1.

Le groupe contrôle recevant les peptides digérés à la pepsine (pB) voit sa capacité moyenne d'inhibition augmenter de 45 à 60% et 59% après 4 et 8 semaines. Cette variation est significative ($p<0.05$ - test T pairé) par rapport au départ, mais stable après 4 semaines.

Pour le groupe recevant les complexes de DnaK-pB, cette capacité passe de 48 à 56% puis 77% sur la même période, cette dernière étant plus importante que dans le groupe contrôle ($p<0.01$ - test T non pairé) et très significative par rapport au temps zéro ($p<0.001$ - T pairé).

De même, les groupes recevant les complexes DnaJ-pB, GroEL-pB et GrpE-pB montrent une élévation très significative après 4 semaines et qui s'accentue encore à la huitième semaine (située bien au dessus de la valeur du groupe contrôle pour les complexes GroEL-pB et GrpE-pB au moment correspondant).

*Inhibition de l'anticorps M7*

Sur la partie droite de la figure 1, sont représentées les évolutions des moyennes d'inhibition (+ écart type) de la fixation de l'anticorps monoclonal M7, par les sérums individuels, pour les différents groupes traités.

Les données chiffrées sont reprises dans le tableau 2.

Le groupe contrôle recevant les peptides de BLG digérés par pepsine (pB) ont une capacité moyenne d'inhibition qui diminue de 70 à 52% et 57% en 4 et 8 semaines. Cette variation est significative ($p<0.01$ - test T pairé), quoique stable après 4 semaines.

Pour le groupe recevant les complexes DnaK-pB, cette capacité se réduit déjà significativement à la quatrième semaine, en passant de 68 à 51%, comme dans le groupe contrôle.

Mais le résultat s'effondre à 17% à la huitième semaine ($p<0.001$ - T pairé), ce qui est nettement inférieur à celui du groupe contrôle pour le prélèvement correspondant ($p<0.01$ - test T non pairé).

L'évolution est parallèle à celle-ci pour le groupe traité avec les complexes de DnaJ-pB.

Pour le groupe recevant des complexes de GroEL-pB, la réduction du pouvoir inhibiteur est d'emblée maximale, atteignant les 28% dès la quatrième semaine, et demeure au même niveau, de 30% à la huitième semaine.

Dans le groupe recevant des complexes GrpE-pB, la réduction du pouvoir inhibiteur est aussi d'emblée maximale, passant de 72 à 22% dès la quatrième semaine, mais semble s'amenuiser ensuite en revenant à un taux moyen de 41%.

*h) Conclusion*

L'administration de peptides issus de la digestion enzymatique d'un antigène majeur du lait, en l'occurrence la bêta-lactoglobuline, sous la forme de complexes liés à des protéines de stress selon l'invention, et par la voie orale, entraîne une modification radicale et très rapide du profil des épitopes reconnus par les anticorps circulants. Ces anticorps sont présents naturellement chez tous les sujets exposés à l'antigène par leur alimentation. Dans un modèle de souris, exposées chroniquement à une quantité faible de l'antigène par cette voie, la dose d'antigène administrée durant un bref laps de temps est très faible, loin en dessous de la quantité ingérée naturellement (estimation 0,25 µg par sujet et par jour de traitement sous forme de complexes et 150 µg par sujet et par jour dans l'alimentation courante).

La rapidité du changement est d'autant plus remarquable que la demi-vie des anticorps sériques, principalement des IgG est de 3 semaines, ce qui signifie qu'à la huitième semaine, il devrait encore subsister le quart des anticorps présents à la fin du traitement de seulement 2 semaines. Toutes les protéines de stress utilisées ont été efficaces. Dans une deuxième expérience avec des complexes DnaK-pB, une tentative de déterminer une dose limite inférieure n'a pas abouti malgré l'usage de doses jusqu'à 10 fois inférieures (0,1 µg / biberon de 100 ml / 3 jours par semaine).

C. Tolérance induite oralement à l'égard d'antigènes d'histocompatibilité majeurs

*1. Modèle expérimental*

Des animaux syngéniques (souris Balbc) reçoivent une préparation protéique dissoute dans leur eau de boisson. Elle contient des fragments d'antigènes d'histocompatibilité de souris syngéniques d'une autre souche, dont elles rejetteraient toute greffe (souris C3H).
L'effet tolérogène est attendu de manière renforcée lorsque ces fragments sont associés à une protéine de stress de bactérie (ici Dnak de E. Coli).
A titre de contrôle, un groupe de souris recevra de la même manière un complexe de Dnak avec des fragments peptidiques, obtenus de façon analogue, à partir de la bêta-lactoglobuline (antigène majeur du lait).
On devrait s'attendre à ce que la sensibilisation orale doit atténuer de façon spécifique la réactivité lymphocytaire à l'égard d'une souche de lymphocytes étrangers du même type que ceux utilisés pour la préparation orale et non vis-à-vis d'une troisième souche non apparentée.

*2. Matériel et méthode*

a) animaux :

3 groupes de 12 souris sont prélevées dans un élevage de souris syngéniques de souche Balbc sont élevées par cages de 6 animaux. Chaque groupe reçoit pendant 2 semaines une des préparations suivantes dans le biberon d'eau de boisson à raison de 3 distributions par semaine (un jour sur deux et pas le week-end) et une dose de 1 $\mu$g de complexe pour 100 ml d'eau :

- un complexe de Dnak-peptides de bêta-lactoglobuline (préparation contrôle)
- une solution de peptides de membrane lymphocytaire splénique de souris C3H digérée à la pepsine (contenant des fragment d'antigènes d'histocompatibilité)
- un complexe de ces peptides associé au Dnak purifié de E. Coli (Stressgen)

b) test de tolérance acquise (in vitro)

Ce test est basé sur une culture lymphocytaire mixte unidirectionnelle.
Les cellules répondeuses sont isolées à partir de la rate des animaux à tester. Les cellules lympho-monocytaires sont obtenues après centrifugation sur gradient de densité avec un mélange de ficoll-isopaque (Pharmacia). Elles sont resuspendues à l'aide de 4 millions de cellules/ml en milieu de culture RPMI 1640 tamponné à l'Hepes et au bicarbonate, supplémenté de 2-mercaptoéthanol, glutamine, géomycine, et 10% de sérum de veau.
Les cellules stimulantes sont obtenues de la même manière de souris de souches différentes au niveau de leur MHC : la souche C3H et une souche domestique (DOM).
Elles sont incubées pendant une heure en présence de mitomycine afin de bloquer leur potentiel de multiplication. Elles sont ensuite resuspendues dans les mêmes conditions que les cellules répondeuses.

Culture lymphocytaire:

Un volume égal de suspension (0,1 ml) de cellules répondeuses et de cellules stimulantes sont mélangés, en 3 exemplaires, dans des puits à fond rond de plaques multipuits de culture en polystyrène pour être ensuite incubées durant 5 jours dans un incubateur à air/$CO_2$ (95/5%; vol/vol), humidifié, à 37,5 °C.
Chaque puits de microculture recevra 2 $\mu$c de thymidine tritiée à 2 C/mM (Amersham) 16 heures avant l'arrêt de la culture qui se réalise à l'aide d'un appareil MASH II qui filtre chaque microculture sur une membrane de fibres de verre qui retient les noyaux cellulaires.
La radioactivité nucléaire de chaque culot, qui reflète l'incorporation de novo de thymidine dans de l'ADN, est mesurée par comptage en scintillation liquide (Packard Tricarb).
Les résultats sont exprimés en Coups Par Minute et représentent la moyenne de 3 échantillons de la même culture à l'échelon individuel.

c) planification expérimentale

Les prélèvements ont lieu à 2 moments différents :

- durant la 3ème semaine qui suit le début de l'administration orale d'une des préparations,
- durant la 7ème semaine.

Les animaux sont sacrifiés en 3 fois par période .

Chaque expérience de culture comporte 2 animaux par groupe traité.

La culture lymphocytaire mixte est réalisée parallèlement :

a) vis-à-vis de cellules mitomycinées d'origine C3H
b) vis-à-vis de cellules mitomycinées d'origine DOM

d) préparation des peptides

Des lymphocytes (20 millions) d'une souche de souris appropriée, sont isolés à partir de la rate. Il s'agit d'un mélange de lymphocyte T et B en quantités approximativement égales et donc porteurs des antigènes de type I et II. Ils sont traités par ultrasons (3 x 10 sec) puis centrifugés à 1000 g pendant 10 minutes. Le surnageant est collecté et recentrifugé de la même façon. Ensuite, le surnageant est centrifugé à 2 reprises à 8000 g. Le dernier surnageant est enrichi en membranes cellulaires et débarrassé de débris de noyau cellulaire et d'appareil de Golgi. Il est soumis ensuite à une digestion par pepsine couplée à des billes d'agarose, à pH 2 en tampon glycine, pendant 1 heure 30 et à 37°C. Après centrifugation douce pour séparer les billes d'agarose, et neutralisation à pH 7 par tampon TRIS, le mélange est filtré sur filtre (Millipore limite 10 kD). Le rendement est de l'ordre 500 µg de peptide (détermination par spectrophotomètrie) dénommé LMp.

Une solution de 50 µg de peptide est mélangée à une solution de 50µg de Dnak (Stressgen) pour former un complexe Dnak-LMp.

Le complexe Dnak avec peptide de bêta-lactoglobuline (Bp) est constitué de la même manière en utilisant des peptides issus de la digestion pepsinique de bêta-lactoglobuline purifiée (cf. supra).

*3. Résultats*

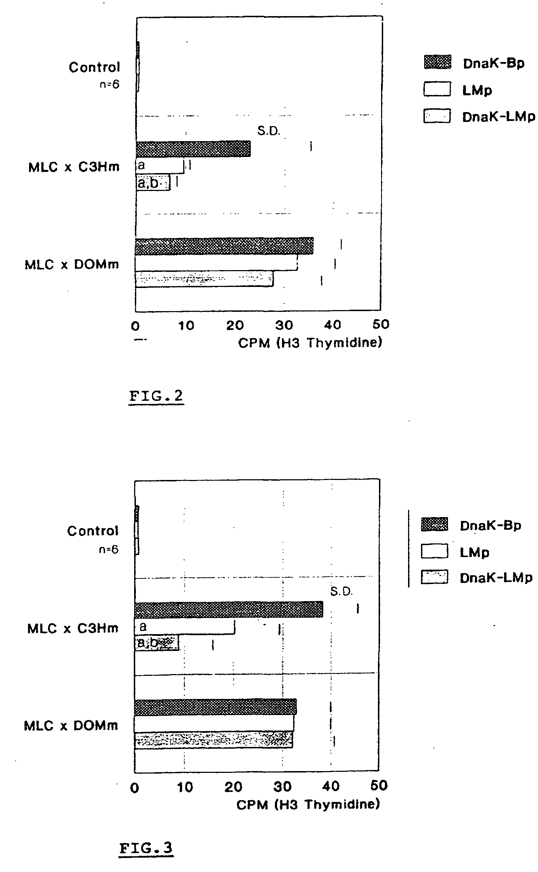

Au bout de 3 semaines de traitement (figure 2)

Le groupe de souris ayant reçu le complexe Dnak-LMp répond le moins bien à la stimulation de cellules C3H mitomycinées (C3Hm).

Ceci est différent (p<0.02; T-test) du groupe qui a reçu le peptide LMp seul, et également celui qui a reçu le complexe témoin Dnak-Bp (p<0.01; T-test).

Il faut cependant noter que l'administration du peptide seul (sans Dnak) a également un effet, puisque ce groupe est significativement moins répondeur que le groupe témoin (p<0.01; T-test).

Toutefois, la spécificité de l'inhibition de réponse est garantie par le fait que la réactivité lymphocytaire des trois groupes est équivalentes à l'égard de cellules mitomycinées d'une troisième souche, non apparentée (DOMm).

Au bout de 7 semaines (figure 3), soit 4 semaines après l'arrêt de l'administration orale

Les différence entre les 3 groupes restent bien marquées. Le groupe traité au Dnak-LMp est le plus inhibé tant à l'égard de celui qui a reçu le peptide membranaire seulement (p<0.02; T-test) que le groupe témoin (p<0.01; T-test).

L'administration du peptide seul permet encore une atténuation de réponse vis-à-vis du groupe témoin (p<0.01; T-test).

La spécificité de la réponse est encore une fois vérifiée par le test parallèle vis-à-vis des cellules mitomycinées d'une souche non apparentée (DOMm) et où les 3 groupes traités différemment réagissent de la même façon.

L'administration de peptides obtenus par digestion pepsiniques de lymphocytes spléniques de souches de souris caractérisées par une incompatibilité dans le système H-2 tant aux niveaux K et D que A-E en quantités extrêmement faibles et pendant deux semaines, a pour effet d'atténuer fortement la réponse inconditionnelle de lymphocytes immuno-compétents, in vitro, qui signe d'habitude cette incompatibilité.

Cette atténuation est renforcée par la présentation de ce type de peptide sous la forme de complexes peptides-Dnak.

Cette atténuation est spécifique et n'atteint en rien la capacité de réponse à l'égard d'une variété différente, sans relation avec la souche utilisée pour la tolérisation.

D. Tolérance des souris syngéniques à l'égard d'une greffe de cellules allogéniques

*1. Modèle et schéma expérimental*

Souches de souris :

- Balbc pour les animaux rendus tolérants
- C3H pour les animaux donneurs de cellules à greffer (allogéniques) et de cellules stimulantes en culture lymphocytaire mixte (MLC)

Elles sont élevées en cages de 6 animaux. Chaque groupe est constitué de 12 animaux par traitement.
Le traitement oral est effectué selon le protocole précédent.

Schéma expérimental :

Complexes administrés dans l'eau de boisson :
jours 0, 2, 4, 7, 9 Greffe allogénique : $20 \times 10^6$ cellules spléniques de C3H en intra-péritonéal :
jour 16 Sacrifice, collecte de rates et mise en culture des cellules spléniques :
15 semaines après la greffe

Détection et numération des cellules allogéniques

*a) Par la présence de cellules porteuses de MHC type II*

Test fonctionnel en culture mixte syngénique bidirectionnelle

Les cellules spléniques de souris traitées et greffées sont mises en culture avec des cellules de souris syngéniques (Balbc) non traitées (naïves).
Normalement, il n'y a aucune réponse proliférative à attendre si le contenu des cellules spléniques d'animaux traités et greffés est uniquement composé de cellules syngéniques.
Par contre, la présence de cellules allogéniques, signalant la prise de greffe in vivo, devrait entraîner une prolifération de type MLC par les cellules dites naïves, non tolérantes, d'autant plus importante que le nombre de cellules étrangères est élevé.
Pour évaluer l'ordre de grandeur de la prise de greffe, une tentative de quantification relative de la réponse est effectuée par référence à une courbe dose/réponse obtenue en ajoutant des quantités connues et croissantes de cellules allogéniques C3H à une quantité identique de cellules naïves et répondeuses ($200 \times 10^3$ cellules/puits).

*b) Par la présence de cellules porteuses de MHC type I*

Numération directe par immunofluorescence en cytofluorométrie de flux en utilisant un anticorps monoclonal de souris spécifique du MHC type I de la souris C3H : H-2 kk (Serotec), couplé à la fluorescéine ou à la phycoérythrine sur une suspension de cellules spléniques.

*2. Résultats*

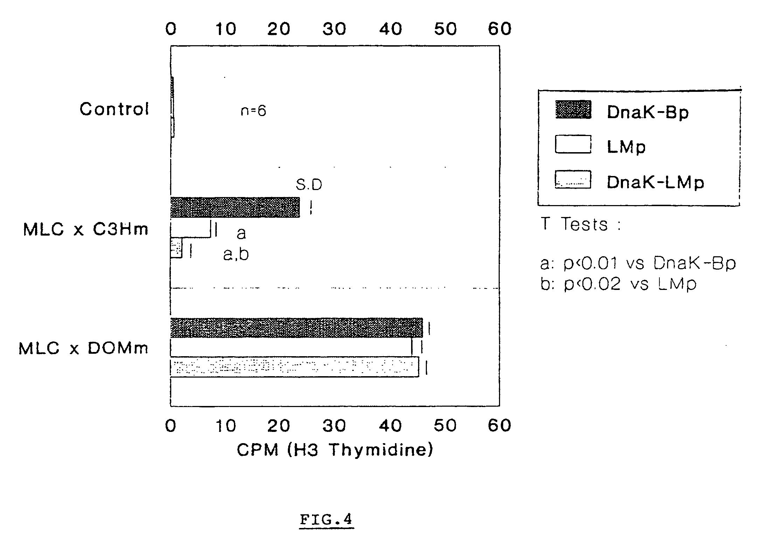

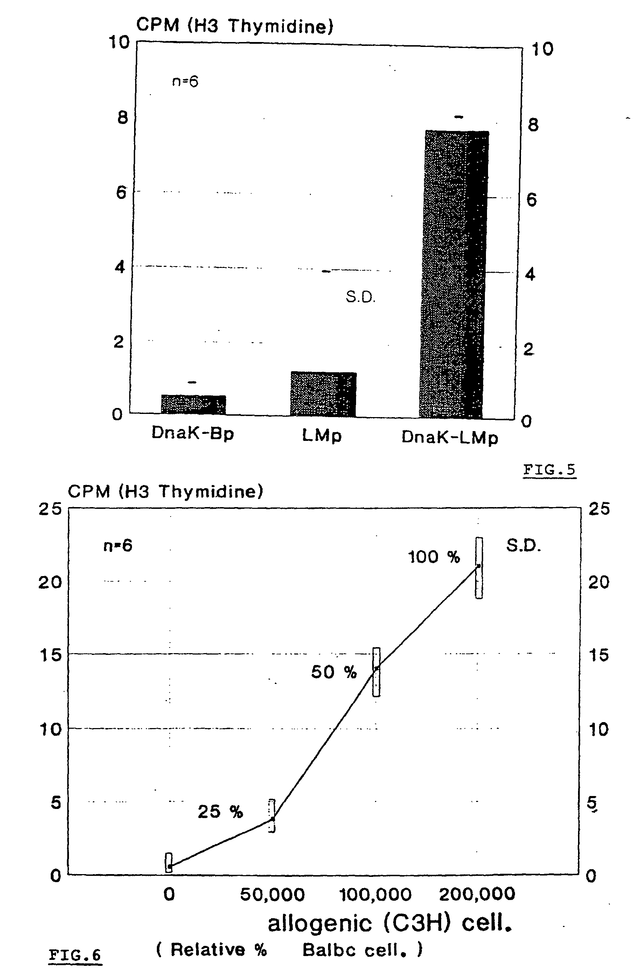

Comme présenté dans la figure 4, 15 semaines après la greffe péritonéale, les cellules spléniques du groupe traité par complexes DnaK-peptides de membranes lymphomonocytaires de souris C3H sont incapables de répondre à la stimulation par cellules C3H mitomycinées. Ceci témoigne de l'induction d'une tolérance allogénique.
Le groupe traité par le peptide seul est également toléré dans une moindre mesure.
Le groupe traité par DnaK-peptide de bêtalactoglobuline n'est pas tolérant du tout.
Par ailleurs, les cultures mixtes stimulées par une autre population allogénique provenant d'une souche histoincompatible différente de C3H, sont toutes semblables. Ceci témoigne de ce qu'aucun traitement n'a altéré ni différencié la capacité de réponse en MLC, et que l'effet du traitement est bien spécifique de la souche de souris dont proviennent les peptides de membranes.
Comme représenté dans la figure 5, on utilise la réponse en MLC de cellules d'animaux ni traités, ni greffés, pour révéler l'existence de cellules étrangères dans un mélange de cellules spléniques d'animaux greffés, qui seraient dont ici d'origine C3H.

Il apparaît que les rates de souris traitées oralement avant la greffe par LMp et DnaK-LMp contiennent des éléments allogéniques puisqu'elles donnent lieu à une réponse proliférative très significativement différente de la réponse du groupe contrôlé traité par un complexe DnaK-peptides irrelevants (bêtalactoglobuline). Ce dernier a une réponse qui ne diffère pas du bruit de fond.

A titre comparatif, une série de cultures mixtes ont été menées parallèlement avec des quantités connues et croissantes de cellules C3H (figure 6). Elles donnent lieu à une réponse proliférative proportionnelle à la quantité de cellules étrangères.

Le niveau de réponse moyen enregistré avec des cellules spléniques d'animaux greffés et tolérisés par complexes DnaK-LMp correspondrait à une teneur d'environ 30% de cellules C3H.

La présence de cellules du type greffé dans la rate est mesurée par immunofluorescence à l'aide d'un anticorps spécifique du MHC I de C3H (H-2kk) (table 5). Le groupe traité par DnaK-LMp en contient 14.7% en moyenne, valeur significativement supérieure à celle des deux autres groupes.

Cette présence d'allo-antigènes n'est observable qu'à la condition de laisser reposer les cellules spléniques d'animaux greffés pendant au moins une suit à 37 °C en l'absence de sérum. ceci suggère qu'il est indispensable de permettre la réexpression de ces antigènes dont la présence serait ainsi modulée in vivo, vraisemblablement par des anticorps anti-H-2kk. Ceci ajoute un autre mécanisme de tolérance de la greffe à la tolérance purement adressée aux cellules T répondeuses.

Table 1

Inhibitions de l'anticorps monoclonal M6 liant le nBLG par des sérums de souris individuels : évolution en fonction du temps en fonction du type de complexe administré oralement.

| | | % d'inhibition de la liaison de M6 | | |
|---|---|---|---|---|
| | | Moyenne | Déviation standard | Nombre de cas |
| Groupe 1 : Contrôle (dBLG seulement) | | | | |
| PREL | 1 | 45.0100 | 8.4146 | 8 |
| PREL | 2 | 60.6750 | 3.9304 | 8 |
| PREL | 3 | 59.6338 | 17.4714 | 8 |
| Groupe 2 : complexes dBLG-DnaK | | | | |
| PREL | 1 | 48.4350 | 7.0540 | 8 |
| PREL | 2 | 56.3675 | 5.6146 | 8 |
| PREL | 3 | 77.0975 | 3.8966 | 8 |
| Groupe 3 : dBLG-DnaJ | | | | |
| PREL | 1 | 23.7350 | 15.3990 | 8 |
| PREL | 2 | 65.7013 | 6.2958 | 8 |
| PREL | 3 | 65.3863 | 4.7270 | 8 |
| Groupe 4 : dBLG-GroEL | | | | |
| PREL | 1 | 24.9538 | 4.7972 | 8 |
| PREL | 2 | 56.0100 | 4.3929 | 8 |
| PREL | 3 | 80.0287 | 1.9401 | 8 |
| Groupe 5 : dBLG-GrpE | | | | |
| PREL | 1 | 37.3313 | 6.4248 | 8 |
| PREL | 2 | 56.6962 | 5.5641 | 8 |
| PREL | 3 | 87.1525 | 7.8731 | 8 |

Table 2

Inhibitions de l'anticorps monoclonal M7 liant le nBLG par des sérums de souris individuels : évolution en fonction du temps en fonction du type de complexe administré oralement.

| | | % d'inhibition de la liaison de M7 | | |
|---|---|---|---|---|
| | | Moyenne | Déviation standard | Nombre de cas |
| Groupe 1 : Contrôle (dBLG seulement) | | | | |
| PREL | 1 | 70.0658 | 3.5541 | 8 |
| PREL | 2 | 52.8224 | 2.3458 | 8 |
| PREL | 3 | 57.0592 | 7.8996 | 8 |
| Groupe 2 : complexes dBLG-DnaK | | | | |
| PREL | 1 | 68.9145 | 2.6698 | 8 |
| PREL | 2 | 51.6908 | 3.0857 | 8 |
| PREL | 3 | 17.2697 | 8.0473 | 8 |
| Groupe 3 : dBLG-DnaJ | | | | |
| PREL | 1 | 78.4276 | 3.4832 | 8 |
| PREL | 2 | 50.8553 | 3.9778 | 8 |
| PREL | 3 | 26.9145 | 3.2069 | 8 |
| Groupe 4 : dBLG-GroEL | | | | |
| PREL | 1 | 73.9671 | 3.1679 | 8 |
| PREL | 2 | 28.5132 | 8.6072 | 8 |
| PREL | 3 | 30.2829 | 14.2174 | 8 |
| Groupe 5 : dBLG-GrpE | | | | |
| PREL | 1 | 72.8355 | 4.7722 | 8 |
| PREL | 2 | 22.2961 | 9.5040 | 8 |
| PREL | 3 | 41.3684 | 6.4331 | 8 |

Table 3

Titres d'anticorps anti (natif) nBLG après transformations logarithmiques : évolution en fonction du temps en fonction du type de complexe administré oralement.

| | | Ln de titres (A. U.) | | |
|---|---|---|---|---|
| | | Moyenne | Déviation standard | Nombre de cas |
| Groupe 1 : Contrôle (dBLG seulement) | | | | |
| PREL | 1 | 3.8526 | .4547 | 8 |
| PREL | 2 | 4.2162 | .3395 | 8 |
| PREL | 3 | 4.2059 | .2946 | 8 |
| Groupe 2 : complexes dBLG-DnaK | | | | |
| PREL | 1 | 3.9738 | .7957 | 8 |
| PREL | 2 | 4.3749 | .6353 | 8 |
| PREL | 3 | 3.2562 | .5057 | 8 |
| Groupe 3 : dBLG-DnaJ | | | | |
| PREL | 1 | 3.7073 | .4435 | 7 |
| PREL | 2 | 4.1348 | .5475 | 8 |
| PREL | 3 | 4.3917 | .5047 | 8 |
| Groupe 4 : dBLG-GroEL | | | | |
| PREL | 1 | 4.3714 | .4215 | 8 |
| PREL | 2 | 3.6419 | .4704 | 8 |
| PREL | 3 | 3.9964 | .2724 | 8 |
| Groupe 5 : dBLG-GrpE | | | | |
| PREL | 1 | 4.1526 | .6401 | 8 |
| PREL | 2 | 4.1739 | .4464 | 8 |
| PREL | 3 | 3.6126 | .4873 | 8 |

Table 4

Différences de l'inhibition entre la liaison des anticorps M6 et M7 au nBLG :

| | | % d'inhibition de la liaison de M6 -% d'inhibition de la liaison de M7 | | |
| --- | --- | --- | --- | --- |
| | | Moyenne | Déviation standard | Nombre de cas |
| Groupe 1 : Contrôle (dBLG seulement) | | | | |
| PREL | 1 | -25.0558 | 9.0035 | 8 |
| PREL | 2 | 7.8526 | 5.6470 | 8 |
| PREL | 3 | 2.5745 | 19.8676 | 8 |
| Groupe 2 : complexes dBLG-DnaK | | | | |
| PREL | 1 | -20.4795 | 8.5253 | 8 |
| PREL | 2 | 4.6767 | 6.1131 | 8 |
| PREL | 3 | 59.8278 | 9.2686 | 8 |
| Groupe 3 : dBLG-DnaJ | | | | |
| PREL | 1 | -54.6926 | 13.8705 | 8 |
| PREL | 2 | 14.8460 | 6.4665 | 8 |
| PREL | 3 | 38.4718 | 5.6903 | 8 |
| Groupe 4 : dBLG-GroEL | | | | |
| PREL | 1 | -49.0134 | 3.9824 | 8 |
| PREL | 2 | 27.4968 | 10.6337 | 8 |
| PREL | 3 | 49.7459 | 14.6732 | 8 |
| Groupe 5 : dBLG-GrpE | | | | |
| PREL | 1 | -35.5043 | 4.8959 | 8 |
| PREL | 2 | 34.4002 | 13.4093 | 8 |
| PREL | 3 | 45.7841 | 8.7931 | 8 |

Table 5

| Cellules allogéniques persistantes (H-2kk +) dans les rates de souris Balbc greffées avec des cellules C3H : | | |
|---|---|---|
| Traitement oral | | |
| DnaK-Bp | Lymphocyte Membrane peptide (LMp) seul | DnaK-LMp |
| 1.0% | 0.9% | 14.2% |
| 1.5 | 2.8 | 25.0 |
| 0.5 | 3.6 | 9.2 |
| 0.5 | 3.7 | 10.4 |
| Moyenne        ET | Moyenne        ET | Moyenne        ET |
| 0.9%        0.5 | 2.7%        1.3 | 14.7%        7.2 |
| T-tests couplés :<br><br>DnaK-LMp / Dnak-Bp :           P = 0.026<br><br>DnaK-LMp / LMp :           p = 0.052 | | |

**REFERENCES**

1. Patterson, R. et al., *Allergy Proc.,* Vol. 15 (5), pp. 239-264 (1994)
2. Ferrick, D.A., *Mak-TW Tolérance and Self-Reactivity in V gamma 1.1 C* gamma *4 transgenic mice*
3. Staines, U. et al., *J. Rheumatol.*, Vol. 54 (3), pp. 145-154 (1995)
4. Polla, B.S. et al., *Clin. Exp. Allergy,* Vol. 23 (7), pp. 548-556 (1993)
5. Healy, A.M. et al., *Ann. N. Y. Acad. Sci.*, Vol 663, pp. 319-330 (1992)
6. Revillard, J.P. et al., *Dev. Biol. Stand.,* Vol. 77, pp. 31-37 (1992)
7. Bousquet, J. et al., *Allergy,* Vol. 49, pp. 31-36 (1994)

**Revendications**

**1.** Composition pharmaceutique et/ou alimentaire comprenant avec un véhicule pharmaceutique et/ou alimentaire adéquat, une protéine de stress et au moins un des épitopes conformationnel ou séquentiel d'une structure antigénique induisant un rejet de greffe ou une réaction allergique ou une réaction auto-immune, ladite protéine bactérienne de stress est une protéine de stress d'une bactérie saprophyte.

**2.** Composition pharmaceutique et/ou alimentaire selon la revendication 1, **caractérisée en ce que** le véhicule pharmaceutique et/ou alimentaire est adéquat pour une administration par voie mucosale ou par voie cutanée.

**3.** Composition pharmaceutique et/ou alimentaire selon la revendication 1 ou 2, **caractérisée en ce que** la protéine de stress et l'épitope forment un complexe.

**4.** Composition pharmaceutique et/ou alimentaire selon l'une quelconque des revendications précédentes, **caractérisée en ce que** l'épitope est obtenu par une hydrolyse avantageusement enzymatique, de préférence à la pepsine, de ladite structure antigénique.

**5.** Composition pharmaceutique et/ou alimentaire selon l'une quelconque des revendications 1 à 4, **caractérisée en ce que** la protéine de stress est une protéine de stress d'E. Coli.

**6.** Composition pharmaceutique et/ou alimentaire selon l'une quelconque des revendications 1 à 4, **caractérisée en ce que** la protéine de stress est choisie parmi le groupe constitué par la protéine de stress GroEL, GrpE, DnaK, ou DNAJ.

**7.** Composition pharmaceutique et/ou alimentaire selon l'une quelconque des revendications précédentes, **caractérisée en ce que** la structure antigénique est un atlergène choisi parmi le groupe constitué par les antigènes majeurs allergiques présents dans le lait, , les antigènes majeurs allergiques présents dans les végétaux, dans les poils d'animaux, dans les venins d'animaux, les antigènes majeurs de la réaction allergique aux acariens, à la mite présente dans la poussière de maison (antigène P1 du Dermatophagoides pteronyssinus), l'antigène majeur de l'Aspergillus fumigatus et l'entérotoxine B staphylococcale (SEB).

**8.** Composition pharmaceutique et/ou alimentaire selon l'une quelconque des revendications précédentes, **caractérisée en ce que** la structure antigénique est un altergène choisl parmi le groupe. constitué par la bêta-lactoglobuline bovine (BLG) issue du lait de vache et les antigènes majeurs allergiques présents dans les pollens et dans le venin de guêpe.

**9.** Composition pharmaceutique et/ou alimentaire selon l'une quelconque des revendications précédentes, **caractérisée en ce que** la structure antigénique est le locus majeur d'histocompatibilité de type I ou II

**10.** Composition pharmaceutique et/ou alimentaire selon l'une quelconque des revendications précédentes **caractérisée en ce qu'**elle comporte un immunosuppresseur, de préférence choisi parmi le groupe constitué par l'azathioprine, les stéroides, les globulines anti-lymphocitaires, la cyclosporine A, la rapamycine, le FK506 (tacrolimus), ou des lymphoklnes, leurs analogues, leurs agonistes ou un mélange d'entre eux.

**11.** Utilisation de la composition pharmaceutique et/ou alimentaire selon l'une quelconque des revendications 1 à 10 pour la préparation d'un médicament destiné à modifier la réponse immunitaire d'un patient vis-à-vis d'une structure antigénique spécifique d'une pathologie liée à un rejet de greffe, une réaction allergique ou une réaction auto-immune.

**12.** Utilisation de la composition pharmaceutique et/ou alimentaire selon l'une quelconque des revendications 1 à 10 pour la préparation d'un médicament
destiné à la désensibilisation d'allergies atopiques ou non atopiques.

**13.** Utilisation de la composition pharmaceutique et/ou alimentaire selon l'une quelconque des revendications 1 à 10 pour la préparation d'un médicament
destiné au traitement ou à la prévention des maladies auto-immunes.

**14.** Utilisation selon la revendication 13 **caractérisée en ce que** les maladies auto-immunes sont les pathologies choisies parmi le groupe constitué par les infections liées au SLE (Systemic Lupus Erythematosus disease), le syndrome Gougerot-Sjôgren (ou pathologie Sjôgren), la polyarthrite rhumatoide, ainsi que les pathologies du type sarcoidosis et osteopenia, spondylarthritis, scleroderma, multiple sclerosis, amyotrophic latéral sclerosis, hyperthyroidism, maladie d'Addison, auto-immune haemolytic anaemia, maladie de Crohn, syndrome de Goddpasture, maladie de Graves, Hashimoto's thyroiditis, idiopathic purpura haemorrhagica, diabètes insulino-dépendants, myasthenia, pemphigus vulgaris, pernicious anaemia, poststreptococcal glomerulonephritis, psoriasis et stérilité spontanée.

**15.** Utilisation de la composition pharmaceutique et/ou alimentaire selon l'une quelconque des revendications 1 à 10 pour la préparation d'un médicament destiné au traitement ou à la prévention de rejets de greffe.

**Patentansprüche**

**1.** Pharmazeutische und/oder Nahrungsmittelzusammensetzung, die zusammen mit einem geeigneten pharmazeutischen und/oder Nahrungsmittefträger ein Stressprotein und wenigstens eines der konformatorischen oder sequentiellen Epitope einer Antigenstruktur umfasst, die eine Transplantatsabstoßung oder eine allergische Reaktion oder eine Autoimmunreaktion induziert, wobei das bakterielle Stressprotein ein Stressprotein eines saprophytischen Bakteriums ist.

**2.** Pharmazeutische und/oder Nahrungsmittelzusammensetzung gemäß Anspruch 1, **dadurch gekennzeichnet, dass** der pharmazeutische und/oder Nahrungsmittelträger für eine transmukosale oder transdermale Verabreichung geeignet ist.

3. Pharmazeutische und/oder Nahrungsmittelzusammensetzung gemäß Anspruch 1 oder 2, **dadurch gekennzeichnet, dass** das Stressprotein und das Epitop einen Komplex bilden.

4. Pharmazeutische und/oder Nahrungsmittelzusammensetzung gemäß einem der vorstehenden Ansprüche, **dadurch gekennzeichnet, dass** das Epitop durch eine Hydrolyse, vorteilhafterweise eine enzymatische Hydrolyse, vorzugsweise mit Pepsin, der Antigenstruktur erhalten wird.

5. Pharmazeutische und/oder Nahrungsmittelzusammensetzung gemäß einem der Ansprüche 1 bis 4, **dadurch gekennzeichnet, dass** das Stressprotein ein Stressprotein von *E. coli* ist.

6. Pharmazeutische und/oder Nahrungsmittelzusammensetzung gemäß einem der Ansprüche 1 bis 4, **dadurch gekennzeichnet, dass** das Stressprotein aus der Gruppe ausgewählt ist, die aus den Stressproteinen GroEL, GrpE, DnaK und DNAJ besteht.

7. Pharmazeutische und/oder Nahrungsmittelzusammensetzung gemäß einem der vorstehenden Ansprüche, **dadurch gekennzeichnet, dass** die Antigenstruktur ein Aliergen ist, das aus der Gruppe ausgewählt ist, die aus den Hauptallergieantigenen, die in Milch vorhanden sind, den Hauptallergieantigenen, die in Pflanzen, in Tierhaaren, in Tiergiften vorhanden sind, den Hauptantigenen der allergischen Reaktion gegen Milben, gegen die Hausstaubmilbe (Antigen P1 von *Dermatophagoides pteronyssinus*), dem Hauptantigen von *Aspergillus fumigatus* und dem Staphylococcus-Enterotoxin B (SEB) besteht.

8. Pharmazeutische und/oder Nahrungsmittelzusammensetzung gemäß einem der vorstehenden Ansprüche, **dadurch gekennzeichnet, dass** die Antigenstruktur ein Allergen ist, das aus der Gruppe ausgewählt ist, die aus Rinder-beta-Lactoglobulin (BLG) aus Kuhmilch und den Hauptallergieantigenen, die in Pollen und in Wespengift vorhanden sind, besteht.

9. Pharmazeutische und/oder Nahrungsmittelzusammensetzung gemäß einem der vorstehenden Ansprüche, **dadurch gekennzeichnet, dass** die Antigenstruktur der Haupthistokompatibilitätskomplex des Typs I oder II ist.

10. Pharmazeutische und/oder Nahrungsmittelzusammensetzung gemäß einem der vorstehenden Ansprüche, **dadurch gekennzeichnet, dass** sie einen Immunsuppressor umfasst, der vorzugsweise aus der Gruppe ausgewählt ist, die aus Azathioprin, Steroiden, Anti-Lymphocyten-Globulinen, Cyclosporin A, Rapamycin, FK506 (Tacrolimus) oder Lymphokinen, ihren Analoga, Agonisten oder einem Gemisch derselben besteht.

11. Verwendung der pharmazeutischen und/oder Nahrungsmittelzusammensetzung gemäß einem der Ansprüche 1 bis 10 zur Herstellung eines Medikaments zur Modifizierung der Immunantwort eines Patienten auf eine spezifische Antigenstruktur mit einer Pathologie, die mit einer Transplantatsabstoßung, einer allergischen Reaktion oder einer Autoimmunreaktion verbunden ist.

12. Verwendung der pharmazeutischen und/oder Nahrungsmittelzusammensetzung gemäß einem der Ansprüche 1 bis 10 zur Herstellung eines Medikaments zur Desensibilisierung von atopischen oder nicht-atopischen Allergien

13. Verwendung der pharmazeutischen und/oder Nahrungsmittelzusammensetzung gemäß einem der Ansprüche 1 bis 10 zur Herstellung eines Medikaments zur Behandlung oder Prävention von Autoimmunkrankheiten.

14. Verwendung gemäß Anspruch 13, **dadurch gekennzeichnet, dass** die Autoimmunkrankheiten Pathologien sind, die aus der Gruppe ausgewählt sind, die aus folgenden besteht: Infektionen, die mit SLE zusammenhängen (systemischer Lupus erythematodes), Sjögren-Syndrom, rheumatoide Polyarthritis, sowie Pathologien des Typs Sarcoidose und Osteopenie, Spondylarthritis, Scieroderma, multiple Sklerose, amyotrophe laterale Sklerose, Schilddrüsenüberfunktion, Addison-Syndrom, autoimmune hämolytische Anämie, Morbus Crohn, Goodpasture-Syndrom, Basedowsche Krankheit, Hashimoto-Syndrom, idiopathische Purpura haemorrhagica, insulinabhängige Diabetes, Myasthenie, Pemphigus vulgaris, perniziöse Anämie, poststreptococcale Glomerulonephritis, Psoriasis und spontane Sterilität.

15. Verwendung der pharmazeutischen und/oder Nahrungsmittelzusammensetzung gemäß einem der Ansprüche 1 bis 10 zur Herstellung eines Medikaments zur Behandlung oder Prävention von Transplantatsabstoßungen.

**Claims**

1. A pharmaceutical and/or food composition comprising, together with a suitable pharmaceutical and/or food vehicle, a stress protein and at least one of the conformational or sequential epitopes of an antigenic structure inducing a graft rejection or allergic reaction or auto-immune reaction, said bacterial stress protein being a stress protein of a saprophytic bacterium.

2. The pharmaceutical and/or food composition according to claim 1, **characterized in that** said pharmaceutical and/or food vehicle is adapted for the transmucosal or transdermal routes of administration.

3. The pharmaceutical and/or food composition according to claim 1 or 2, **characterized in that** the stress protein and the epitope form a complex.

4. The pharmaceutical and/or food composition according to any of the preceding claims, **characterized in that** the epitope is obtained by hydrolysis, advantageously enzymatic hydrolysis, preferably by pepsin, of said antigenic structure.

5. The pharmaceutical and/or food composition according to any of claims 1 to 4, **characterized in that** said stress protein is an *E. coli* stress protein.

6. The pharmaceutical and/or food composition according to any of claims 1 to 4, **characterized in that** said stress protein is selected from the group consisting of the GroEL, GrpE, DnaK and DnaJ stress proteins.

7. The pharmaceutical and/or food composition according to any of the preceding claims, **characterized in that** said antigenic structure is an allergen selected from the group consisting of the major allergic antigens present in milk, the major allergic antigens present in plants, in animal hairs, in animal venoms, the major antigens of the allergic reaction against mites, against the house dust mite (antigen P1 of *Dermatophagoides pteronyssinus*), the major antigen of *Aspergillus fumigatus*, and staphylococcal enterotoxin B (SEB).

8. The pharmaceutical and/or food composition according to any of the preceding claims, **characterized in that** said antigenic structure is an allergen selected from the group consisting of bovine beta-lactoglobulin (BLG) from cow's milk and the major allergic antigens present in pollen and in wasp venom.

9. The pharmaceutical and/or food composition according to any of the preceding claims, **characterized in that** said antigenic structure is type I or II major histocompatibility complex.

10. The pharmaceutical and/or food composition according to any of the preceding claims, **characterized by** comprising an immunosuppressor, preferably selected from the group consisting of azathioprine, steroids, anti-lymphocyte globulins, cyclosporin A, rapamycin, FK506 (tacrolimus), or lymphokines, their analogues, their agonists or a mixture thereof.

11. Use of the pharmaceutical and/or food composition according to any of claims 1 to 10 for the preparation of a medicament for modifying the immune response of a patient to a specific antigenic structure related to graft rejection, an allergic reaction or an auto-immune reaction.

12. Use of the pharmaceutical and/or food composition according to any of claims 1 to 10 for the preparation of a medicament for the desensitization of atopic or non-atopic allergies.

13. Use of the pharmaceutical and/or food composition according to any of claims 1 to 10 for the preparation of a medicament for the treatment or prevention of auto-immune diseases.

14. The use according to claim 13, **characterized in that** said auto-immune diseases are pathologies selected from the group consisting of infections related to SLE (systemic lupus erythematosus disease), Sjögren's syndrome, rheumatoid polyarthritis, as well as pathologies of the sarcoidosis and osteopenia types, spondylarthritis, scleroderma, multiple sclerosis, amyotrophic lateral sclerosis, hyperthyroidism, Addison's disease, auto-immune hemolytic anemia, Crohn's disease, Goodpasture syndrome, Graves' disease, Hashimoto's disease, idiopathic purpura hemorrhagica, insulin-dependant diabetes, myasthenia, pemphigus vulgaris, pernicious anemia, poststreptococcal glomerulonephritis, psoriasis and spontaneous infertility.

15. Use of the pharmaceutical and/or food composition according to any of claims 1 to 10 for the preparation of a medicament for the treatment or prevention of graft rejections.

FIG.1

■ Monocl.#6 inhibition  ▨ Monocl.#7 inhib. + SD

FIG.2

FIG.3

FIG.4

FIG.5

FIG.6